# EUROPEAN PATENT APPLICATION

(11) **EP 1 712 208 A2**
(43) Date of publication of application: **18.10.2006**
(21) Application number: 06007698.1
(22) Date of filing: 12.04.2006
(51) Int. Cl.: A61F 7/12, A61B 18/02, A61B 18/20, A61N 5/06, A61B 18/00, A61B 18/24, A61N 5/04

(54) **Intraluminal heat treatment of a lumen combined with cooling of tissue proximate the lumen**

(30) Priority: 14.04.2005 US 671098 P; 10.01.2006 US 328466
(71) Applicant: Asah Medico A/S, 2650 Hvidovre (DK)
(72) Inventor: Balle-Petersen, Olav, 2990 Nivå (DK); Hansen, Dennis Bo, 2100 Copenhagen (DK); Asaii, Bjarne, 2630 Taastrup (DK)
(74) Representative: Jakobsen, Gert Hoey

(57) **Abstract**

The present invention relates generally to a method and an apparatus for intraluminal heat treatment of a lumen in combination with cooling of tissue proximate the lumen. In particular an apparatus for intraluminal energy treatment is provided, comprising an energy source for insertion into a lumen of a body and having an output end for energy-emission within the lumen, and a cooling device with a cooling member adapted to be positioned on the skin surface proximate the lumen along a part of the lumen to be treated for cooling tissue proximate that part of the lumen.

## Description

The present invention relates generally to a method and an apparatus for intraluminal heat treatment of a lumen in combination with cooling of tissue proximate the lumen.

A number of ways for treatment of vascular diseases like e.g. thrombose or varicose are available. The least invasive treatments use an energy delivery arrangement for providing energy intraluminally to change the fluid flow dynamics of the blood vessel.

In for example US 5,053,033 to Clarke and US 6,398,777 to Navarro et al. laser energy is delivered endoluminally. Spanish Patent No. ES2132028 to Salat et al. describes an endoluminal electro-coagulator for varicose vein operations. US 6,613,045 to Laufer et al. describes endoluminal provision of radio frequency energy, microwave energy, or thermal energy.

Local anaesthesia and sometimes even general anaesthesia is used during intraluminal treatment, e.g. during varicose vein surgery.

It is an object of the present invention to provide a method and an apparatus for intraluminal heat treatment of a lumen in combination with cooling of tissue proximate the lumen during intraluminal treatment so that the need for anaesthesia is eliminated.

It is a further object of the present invention to provide a method and an apparatus for cosmetic treatment of varicose veins by intraluminal heat treatment of the varicose veins in combination with cooling of tissue proximate the lumen during intraluminal treatment so that the need for anaesthesia is eliminated.

It is a still further object of the present invention to provide a method and an apparatus for cooling the skin of a human during intraluminal heat treatment, significantly reducing or eliminating influence of the treatment on tissue adjacent the target tissue subjected to the heat treatment.

According to a first aspect of the invention, the above-mentioned and other objects are fulfilled by provision of a method for intraluminal heat treatment of a human, comprising the steps of
locating a part of a lumen to be treated, typically by visual inspection of the appearance of the human's body,
positioning a cooling member on the skin surface proximate the lumen along a part of the lumen to be treated for cooling tissue proximate that part of the lumen,
inserting an energy-emitting member into the lumen, and
moving the energy-emitting member along the longitudinal extension of the part of the lumen to be treated for heating of the lumen wall by energy emission.

According to a second aspect of the invention, the above-mentioned and other objects are fulfilled by provision of a method for cosmetic treatment of varicose veins, comprising the steps of
locating a part of a lumen of the varicose veins to be treated, typically by visual inspection of the appearance of the human's body,
positioning a cooling member on the skin surface proximate the lumen along a part of the lumen to be treated for cooling tissue proximate that part of the lumen,
inserting an energy-emitting member into the lumen, and
moving the energy-emitting member along the longitudinal extension of the part of the lumen to be treated for heating of the lumen wall by energy emission.

According to a third aspect of the invention, the above-mentioned and other objects are fulfilled by provision of an apparatus for intraluminal energy treatment, comprising
an energy source for insertion into a lumen of a body and having an output end for energy-emission within the lumen, and
a cooling device with a cooling member adapted to be positioned on the skin surface proximate the lumen along a part of the lumen to be treated for cooling tissue proximate that part of the lumen.

Throughout this document the terms intraluminal treatment or endoluminal treatment mean a treatment during which energy is emitted within a lumen of a human for treatment of tissue forming a lumen wall or treatment of tissue resident in or proximate to the lumen.

For example, varicose veins may be treated by laser light. During treatment an optical fibre coupled to a laser is introduced into the vein lumen to deliver intraluminal laser energy within the lumen from the output end of the fibre.

For example, the fibre may be operatively coupled to a laser emitting energy in the wavelength range from 500 nm to 1800 nm. Energy in this wavelength range is effectively absorbed by haemoglobin in the blood whereby the blood is heated and the heated blood in turn heats the vein wall.

Also in this wavelength range, human fatty tissue has lower energy absorption than coliagen so that energy in this wavelength range passing the vein wall will do little damage to the tissue surrounding the vein.

Heating of the vein wall causes fibrosis of the vein wall leading to a decrease in the diameter of the vein and removal of the vein during the subsequent healing. The amount of fibrosis in the vein wall is determined by the amount of laser energy delivered to the wall. Preferably, the vein wall is damaged to an extent that the subsequent fibrosis causes the vein to collapse.

Typically, the optical fibre is inserted through an angiocatheter placed percutaneously into the vein to be treated.

In another example, thrombose is treated by removal of material in the lumen thereby enlarging the lumen.

Thus, the energy source may comprise a light source for emission of treatment light, such as a laser, such as a laser having a wavelength ranging from 800 nm to 820 nm, or from 930 nm to 950 nm, or from 970 nm to 990 nm, a 810 nm diode laser, a 940 nm diode laser, a 980 nm diode laser, etc., and an optical fibre coupled to the laser source for insertion into the lumen and having an output end for emission of the treatment light.

Preferably, the power of the laser light ranges from 5 W to 20 W, and more preferred from 8 W to 15 W.

Preferably, laser light is delivered in pulses having duration of less than 2 seconds, such as in the range from 0.5 to 1.5 seconds.

Typically, the energy is emitted in pulses, emitted upon actuation by the operator of the apparatus, e.g. actuation of a foot pedal. Upon pulse emission, the fibre end is moved from 2 mm to 10 mm, preferably 3 mm - 4 mm, along the lumen before a new pulse emission. At the start of energy emission, dissipated heat may cause expansion of the lumen whereby the detected energy level at the skin increases. This may be utilized for recording of the amount of energy delivered within the lumen during the entire treatment.

The laser light may be emitted continuously in which case the fibre may be moved approximately 5 mm pr. second.

Alternatively, the energy source may comprise a radio frequency source, such as a bipolar generator, and a set of electrodes coupled to the generator for insertion into the lumen and having an output end for creating a heated region of the lumen wall. Alternatively, a microwave source or a heat source may be used.

Energy emitted within the lumen may inadvertently pass the lumen wall and be absorbed by tissue surrounding the lumen thereby heating the tissue proximate the lumen. In order to keep the temperature of tissue proximate the treated lumen sufficiently low for damaging of this tissue to be avoided, the tissue is cooled by a cooling member positioned on the skin surface proximate the lumen along a part of the lumen to be treated. Preferably, cooling is performed before, during and after emission of energy within the lumen so that a damaging temperature increase of tissue proximate the treated lumen is effectively avoided. However, cooling may be applied before the energy is emitted, simultaneous with the energy being emitted, after the energy has been emitted, or in any combination hereof as appropriate.

Preferably, the cooling member has a size sufficiently large to cool tissue proximate the lumen without being moved during the treatment whereby effective cooling of influenced tissue is ensured during the entire treatment. Further, movement of the cooling member adds waiting time to the treatment until the tissue at the new position of the member has been cooled sufficiently for the treatment to continue. Such waiting time is avoided with a sufficiently large cooling member.

Preferably, the contact surface of the cooling member has a width of at least 1 cm, preferably at least 3 cm, more preferred at least 5 cm, and a length of at least 5 cm, preferably least 6 cm, more preferred at least 10 cm. A rectangular area of this size permits cooling to be applied to an appropriate area proximate the treated lumen. In a preferred embodiment, the cooling member has an area sufficient for substantially covering the front surface of the thigh of a human.

In a preferred embodiment of the invention, the cooling member is a flexible cooling member capable of comfortably fitting onto a curved surface of a human.

The cooling member may have a compartment for accommodation of a cooling fluid. The cooling member may further have an input and an output for cooling fluid interconnecting the compartment and a refrigerator for recirculation and cooling of the cooling fluid. The cooling fluid is preferably water or a water/alcohol mixture, preferably refrigerated. The temperature of the cooling fluid is preferably controlled in such a way that the contact surface of the cooling member has a temperature ranging from -1 °C to + 15 °C, preferably from -1 °C to +10°C.

In a preferred embodiment, a flexible bag constitutes the cooling member with channels for the flow of cooling fluid Preferably the bag is made of soft PVC. Further, the compartment may be divided into a meander shaped flow channel by internal walls forming a flow path for the cooling fluid from the input to the output. The flexibility of the plastic bag enables it to substantially fit and contact the skin surface of the human's body proximate the lumen to be treated without compressing the tissue undemeath the bag. Hereby, an improved cooling of the entire surface is obtained and it is possible to apply the cooling member without compressing the lumen.

In another embodiment, the cooling device comprises an opening, such as a spray nozzle, for emission of a cooling spray, e.g. an air flow, a cryogenic spray, etc., onto the skin proximate to the output end.

In yet another embodiment, the cooling device comprises an opening, such as a spray nozzle, for emission of a cooling spray, such as an air flow, a cryogenic spray, etc., onto a cooling member having a contact surface adapted to be in contact with the skin during treatment.

Monitoring of the positioning of the output end of the optical fibre may be performed by emission of visible light from the output end, or by utilization of ultrasonic imaging.

Ultrasound imaging, or Doppler ultrasound imaging, may be used to visualize on a display unit, such as a CRT screen, a LCD screen, etc., the position of the fibre output end, e.g. during insertion of the fibre into the lumen, such as a vein, and before and after movement of the fibre in the lumen during treatment to verify correct positioning of the output end. Further, the lumen and the lumen walls may be visualized on the display unit, e.g. to monitor changes of lumen shape or dimensions before and after emission of treatment light to verify the effect of the treatment.

By emission of visible light, the visible output light can be seen through the skin and may be emitted simultaneously with light of another wavelength. Thus, the apparatus according to the invention may further comprise a light source for emission of visible light that is coupled to the optical fibre whereby the visible light is output from the output end of the optical fibre. The visible light emitted by the output end is used for detection through the skin of the position of the output end. An operator of the apparatus may determine the position by observing and locating the visible spot on the skin of the human to be treated. The visible spot indicates the skin position closest to the output end within the lumen.

The apparatus may also comprise a detector, such as a photo detector, etc, for detection of emitted visible light whereby the amount of emitted treatment energy may by recorded. At the start of energy emission, dissipated heat may cause expansion of the lumen whereby the level of visible light at the skin increases. This may be utilized for recording of the number of energy pulses delivered within the lumen and thereby recording of the total amount of energy delivered during the entire treatment.

The cooling member may further comprise a window for transmission of energy emitted by the output end facilitating detection of the position of the output end when the cooling device is positioned at the skin proximate the output end. In a preferred embodiment, the window is transparent to visible light, such as light with a wavelength within the range from 600 nm to 700 nm, or 500 nm to 550 nm, so that the operator is allowed to observe the light penetrating the skin beneath the cooling member during treatment.

The window may constitute the contact surface or may form a part of the contact surface.

In an embodiment, the window forms an upper and a lower wall of the compartment, the lower wall constituting the contact surface being in contact with skin during treatment and the upper wall facing away from the skin during treatment so that energy may be transmitted through the walls and the cooling medium in the compartment during treatment facilitating observation of the treatment area.

In another embodiment, the window constitutes a cooling member in contact with the compartment for transmission of heat from the skin to the cooling medium and allowing energy to be transmitted through the window during treatment facilitating observation of the treatment area.

The window may be made from a material having a crystalline structure, such as sapphire or quartz, e.g. a synthetic sapphire. The thermal conductivity of the window is preferably at least 25 W/m°C. The cooling medium may be thermally coupled to the window along the edges of the window, it may flow through channels in the window, or it may flow over the surface of the window.

The window may be provided with a distance indicator permitting an operator of the apparatus to observe the distance of retraction or insertion of the fibre output end within the lumen by observation of visible light emitted from the output end and transmitted through the lumen wall and tissue proximate the lumen to the skin surface.

The detector may further be adapted for sensing change in the intensity of a substantially monochromatic radiation.

In a preferred embodiment, the member contact surface has a recessed section whereby the lumen may remain uncompressed or partly compressed when said contact surface is pressed against the skin surface proximate the lumen.

It is an important advantage of the present invention that the need for local anaesthesia of treatment sites is significantly reduced or eliminated.

It is another important advantage of the present invention that influence of the treatment on tissue adjacent target tissue to be treated by the emitted energy is significantly reduced or eliminated.

The above and other features and advantages of the present invention will become more apparent to those of ordinary skill in the art by describing in detail exemplary embodiments thereof with reference to the attached drawings in which:
- Fig. 1: schematically illustrates a cooling member of an apparatus according to the present invention,
- Fig. 2: schematically illustrates an apparatus according to the present invention for spraying a cooling medium onto the skin at the treatment site,
- Fig. 3: schematically illustrates an apparatus according to the present invention for spraying a cooling medium onto a cooling member,
- Fig. 4: schematically illustrates a cooling member of an apparatus according to the present invention having a recess for reducing compression of the lumen to be treated,
- Fig. 5: is a perspective view of another cooling device in accordance with the present invention, and
- Fig. 6: is a perspective view of a cooling device comprising a cooling member constituted by a flexible bag in accordance with the present invention

The present invention will now be described more fully hereinafter with reference to the accompanying drawings, in which exemplary embodiments of the invention are shown. The invention may, however, be embodied in different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

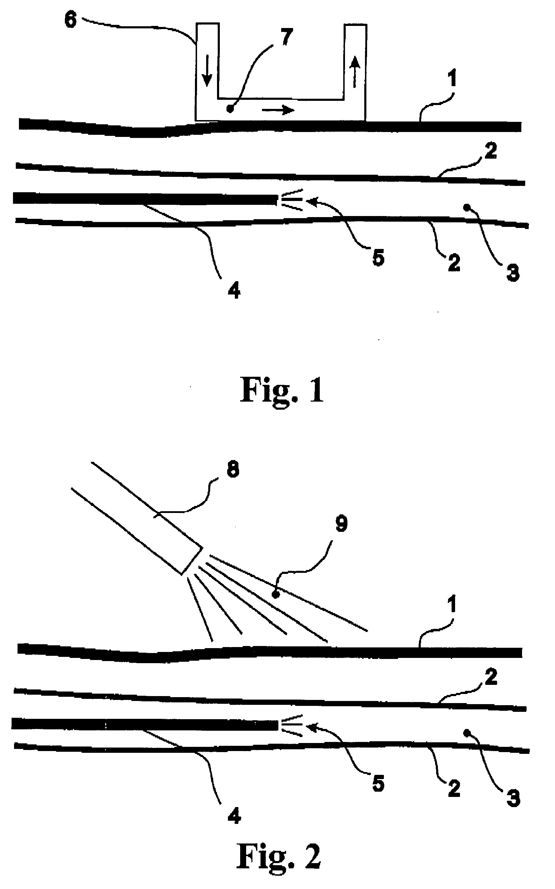

Fig. 1 schematically illustrates a first embodiment of the invention for treatment of varicose veins. The apparatus comprises a laser (not shown) coupled to an optical fibre 4 for emission of light 5 at its output end. The optical fibre is positioned inside the lumen 3 of the vein 2, e.g. the greater saphenous vein, to be treated. A cooling member 6 is positioned in contact with the skin 1 of the human for cooling the skin proximate the output end and the intervening tissue. The cooling member includes a compartment for accommodation of a cooling medium 7. The cooling compartment is connected to cooling channels, which in turn are connected to a refrigerator for recirculation and cooling of the cooling medium. The cooling medium may be a gas, such as a non ozone-depleting gas, or a liquid, such as a water/alcohol mixture, etc. The temperature of the cooling medium is controlled in such a way that the contact surface of the cooling member has a temperature ranging from -1°C to +10°C. The cooling member further comprises a window for transmission of light emitted by the output end facilitating detection of the position of the output end when the cooling member 6 is positioned at the skin 1 proximate the output end so that the operator is allowed to observe the skin beneath the cooling member during treatment. The window forms an upper and a lower wall of the compartment, the lower wall constituting the contact surface being in contact with skin during treatment and the upper wall facing away from the skin during treatment so that light is transmitted through the walls and the cooling medium in the compartment during treatment facilitating observation of the treatment area. In the event that the treatment light is not visible, a further light source (not shown) is coupled to the optical fibre 4 for additional emission of visible light from the output end for transmission through the window and the cooling medium 7 facilitating observation of the position of the output end within the lumen in relation to the skin.

The window may further comprise ruler like distance indications, e.g. in the form of at least two marks on the window having a certain distance. Such distance indications permits the operator to control the distance of retraction or insertion of the fibre output end within the lumen by observation of visible light emitted from the output end and transmitted through the lumen wall and tissue proximate the lumen to the skin surface.

A second embodiment of the invention is schematically illustrated in Fig. 2. The illustrated embodiment of the invention comprises a spray nozzle 8 for emission of a cooling spray 9 onto the skin proximate the endoluminal treatment site, i.e. the position of the output end, for cooling of the skin proximate the output end and the intervening tissue. The cooling medium may be e.g. cold air, cold gas, cold vapour, or cryogenic liquid drops. The cooling medium has to be added in a sequence of bursts or as a steady stream to maintain the desired temperature of the skin. The temperature of the cooling medium is controlled so that the skin of the human is cooled to a temperature between -1°C and +10°C

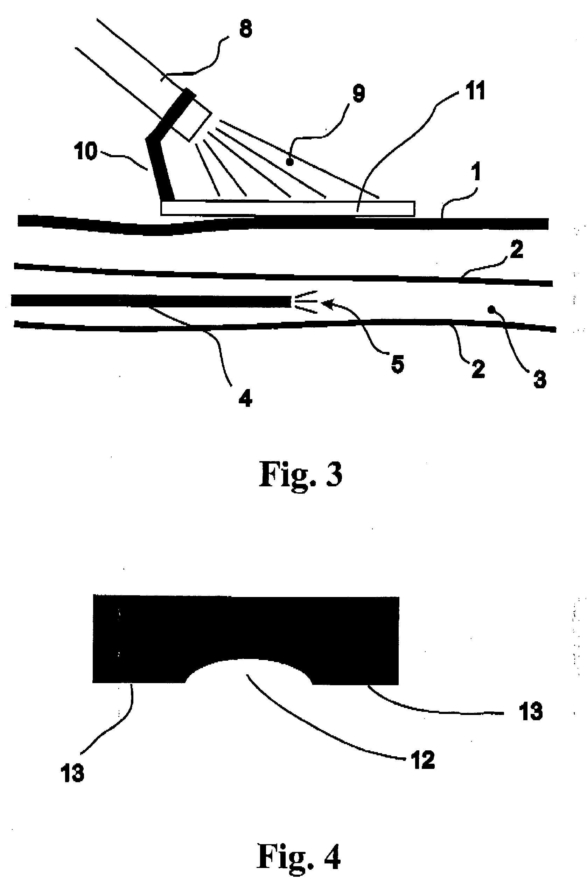

The embodiment schematically illustrated in Fig. 3 corresponds to the embodiment of Fig. 2. However, the illustrated embodiment further comprises a cooling member 11 positioned in contact with the skin of the human. The spray nozzle 8 emits the cooling spray 9 onto the cooling member 11 thereby indirectly cooling the skin. The cooling medium may be cold air, cold gas, cold vapour, or cryogenic liquid drops. The temperature of the cooling medium is controlled so that the skin of the human is cooled to a temperature between -1°C and +10°C.

Fig. 4 schematically illustrates a cooling member 13 of an apparatus according to the present invention having a recess 12 for reducing compression of the lumen to be treated. For example, during treatment of a blood vessel, the recessed section prevents pressing blood out of the vessel, hence the blood is allowed to stay at the treatment site and absorb and conduct the energy 5 emitted in the lumen 3 of the blood vessel 2. Preferably, the recessed section extends from one edge of the cooling member to the opposite edge and has a width of between 5 mm and 10 mm, and a depth of between 5 mm and 10 mm. The cooling members shown in Figs. 1 and 3 may comprise such a recess 12.

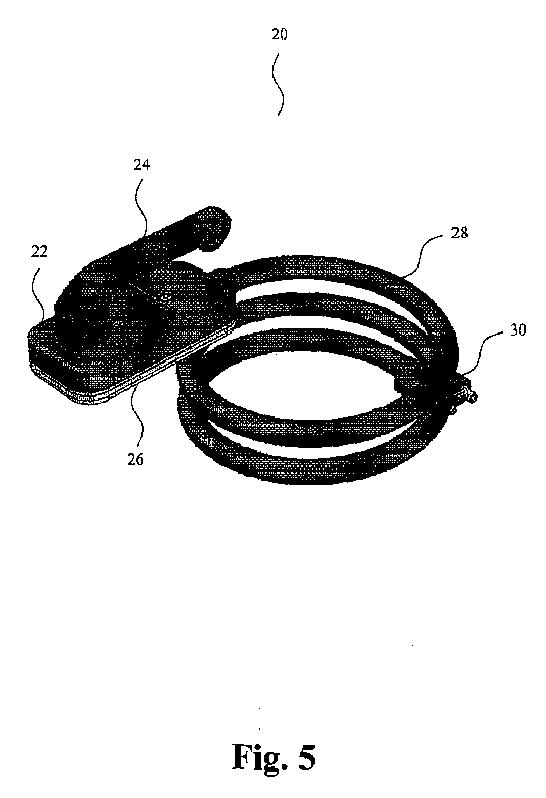

Fig. 5 is a perspective view of another cooling device 20 according to the invention for treatment of varicose veins. The cooling device 20 comprises a cooling member 22 with a handle 24 for manual positioning of the cooling member 22 on the skin surface of a human proximate the lumen to be treated. The cooling member 22 has a cooling surface 26 that is positioned in contact with the skin of the human for cooling the skin above the veins to be treated and the intervening tissue. The cooling member 20 includes a compartment (not visible) for accommodation of a cooling fluid. The cooling compartment is connected to input and output cooling channels residing in the hose 28, which in turn are connected with connector 30 to a refrigerator (not shown) for recirculation and cooling of the cooling fluid. The cooling fluid is preferably a waterlalcohol mixture. The temperature of the cooling fluid Is controlled in such a way that the contact surface 26 of the cooling member 22 has a temperature ranging from - 1°C to +10°C. Preferably, the cooling member 22 is capable of cooling the front surface of the human's thigh without a requirement of moving the member 22 during treatment so that the treatment is not delayed by such movement that would include waiting time until the tissue at the new position of the member 22 has been cooled sufficiently for the treatment to continue.

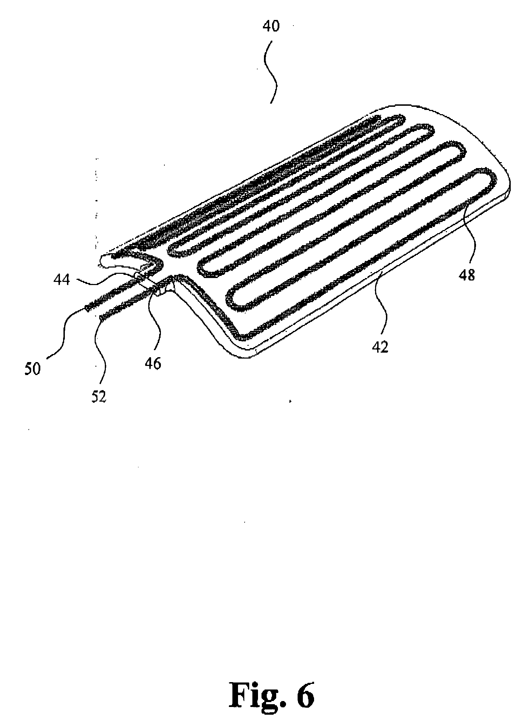

Fig. 6 is a perspective view of a preferred embodiment of a cooling device 40 comprising a cooling member constituted by a flexible bag 42. The bag 42 has an input 44 and an output 46 for cooling fluid and internal walls dividing the internal volume of the bag 42 into a meander shaped flow channel 48 forming a flow path for the cooling fluid from the input 44 to the output 46. The input 42 and the output 46 are connected through respective hoses 50, 52 to a refrigerator (not shown) for recirculation and cooling of the cooling fluid. The cooling fluid is preferably water or a water/alcohol mixture. The temperature of the cooling fluid is controlled in such a way that the contact surface of the bag 42 has a temperature ranging from -1 °C to +10°C. The bag 42 is capable of cooling the front surface of the human's thigh without a requirement of moving the bag 42 during treatment. Hereby, treatment delays are avoided since movement would include waiting time until tissue at the new position of the bag 42 has been cooled sufficiently for the treatment to continue.

The material of the bag may be transparent. The bag 42 is made of soft PVC. The flexibility of the plastic bag 42 enables it to substantially fit the contour of the human's skin surface, e.g. the skin surface of a thigh along substantially the entire front surface of the thigh, without necessarily compressing the tissue underneath the bag 42.

## Claims

1. An apparatus for intraluminal energy treatment, comprising
an energy source for insertion into a lumen of a body and having an output end for energy-emission within the lumen, and
a cooling device with a cooling member adapted to be positioned on the skin surface proximate the lumen along a part of the lumen to be treated for cooling tissue proximate that part of the lumen.

2. An apparatus according to claim 1, wherein the cooling member has a recess to be positioned proximate the lumen to be treated thereby substantially avoiding compression of the lumen during treatment.

3. An apparatus according to claim 1 or 2, wherein the cooling member has a cooling surface that is adapted to substantially cover the front surface of a human thigh.

4. An apparatus according to any of the preceding claims, wherein the cooling device comprises a flexible cooling member that is adapted for comfortably fitting onto a curved surface of a human.

5. An apparatus according to claim 4, wherein the flexible cooling member has an input and an output for cooling fluid and internal walls dividing the internal volume of the cooling member into a meander shaped flow channel forming a flow path for the cooling fluid from the input to the output.

6. A method for cosmetic treatment of varicose veins, comprising the steps of
locating a part of a lumen of the varicose veins to be treated,
positioning a cooling member on the skin surface proximate the lumen along a part of the lumen to be treated for cooling tissue proximate that part of the lumen,
inserting an energy-emitting member into the lumen, and
moving the energy-emitting member along the longitudinal extension of the part of the lumen to be treated for heating of the lumen wall by energy emission.
